# EUROPEAN PATENT APPLICATION

(11) **EP 4 105 312 A1**
(43) Date of publication of application: **21.12.2022**
(21) Application number: 21180081.8
(22) Date of filing: 17.06.2021
(51) Int. Cl.: C12M 1/00, C12M 1/42, C12N 13/00, G16B 40/00

(54) **METHOD AND SYSTEM FOR THE IDENTIFICATION OF OPTIMIZED TREATMENT CONDITIONS**

(71) Applicant: Bühler AG, 9240 Uzwil (CH)
(72) Inventor: Buchmann, Leandro, 8408 Winterthur (CH)
(74) Representative: Hepp Wenger Ryffel AG

(57) **Abstract**

The present invention is related to a method for the identification of optimized conditions for treating cells with electric pulses for targeted inactivation, the extraction of bioactive compounds, and the stimulation of cell growth and/or cellular compounds, comprising the steps of treating samples comprising cellular material under at least one condition, preferably two or more different conditions, analysing the results of the treatment in step a) for each of the applied different conditions, and identifying suitable conditions from the analysis of step b). The present invention is furthermore related to a system (1) for performing said method, as well as to a method for treating cells for targeted inactivation, the extraction of bioactive compounds, and the stimulation of cell growth and/or cellular compounds, employing the above identified suitable conditions.

## Description

The present invention is related to a method and a system for the identification of optimized treatment conditions for medical, environmental, food applications, and bio-based industries (including yeast, lactobacilli, algae, and cell tissue production systems, in particular including targeted inactivation, the extraction of bioactive compounds, and the stimulation of cell growth and/or cellular compounds.

It is known that prokaryotic and eukaryotic cells are influenced by the action of electric fields. Stimulation of cell growth, as well as cell death, inactivation of microorganisms, or specific extraction of cell constituents can occur, depending on the applied electric field strength (e.g. Buchmann L and Mathys A (2019), Perspective on Pulsed Electric Field Treatment in the Bio-based Industry, Front. Bioeng. Biotechnol. 7:265, doi: 10.3389/fbioe.2019.00265) .

EP-2 308 969 B1 describes a PEF (pulsed electric field) method where a cell material suspended in an electrically conductive liquid, the cell material being positioned between two electrodes, by exposure of 1 to 100 electric field strength pulses, such that a voltage increase takes place between the two electrodes of 10% to 90% of a target voltage of the electric field strength pulses within a period of 0.1 to 100 ns, the electric field strength pulses have a pulse duration of 5 ns to 5000 ns, and the electric field strength pulses, upon reaching the target voltage, have an electric field strength of 0.5 kV/cm to 50 kV/cm, showed an accelerated cell proliferation and/or increased cell constituents.

A drawback of this method is that it may take up to several weeks to obtain a feedback as to whether the applied treatment parameters have been suitable. That is, after a treatment in a device such as described in EP-2 308 969 B1 has been performed, it takes some time until it can be evaluated whether the treated cellular material has, for example, grown in a desired manner.

It was the problem underlying the present invention to shorten the time requirements associated with the prior art method, thereby improving the efficiency and economy of such a method.

The above problem has been solved by the present invention.

In detail, the present invention is related to a method for the identification of optimized conditions for treating cells with electric pulses for targeted inactivation, the extraction of bioactive compounds, and the stimulation of cell growth and/or cellular compounds, comprising the steps of:
a) treating samples comprising cellular material under at least one condition, preferably two or more different conditions,
b) analysing the results of the treatment in step a) for each of the applied condition(s),
c) identifying suitable conditions from the analysis of step b) .

According to the present invention, a screening for suitable conditions for treating cells with electric pulses is performed. This screening is performed in a time-efficient manner and thus allows for a fast establishment of suitable treatment conditions for a method for treating cells for targeted inactivation, the extraction of bioactive compounds, and the stimulation of cell growth and/or cellular compounds.

According to a first preferred embodiment of the present invention, step a) of the above method, i.e. the step of treating samples comprising cellular material under different conditions, is performed in a system where the samples flow through a treatment area to which varying treatment conditions are applied, and for each of the applied treatment condition a probe is analysed in step b) of the above method, i.e. the step of analysing the results of the treatment in step a) for each of the applied different conditions.

In step a), samples of cellular material having the same composition are treated under varying treatment conditions.

The treatment area through which the sample flows and to which varying treatment conditions are applied, may be a single-flow device. This is a device through with a liquid material flows in one direction. Generally, this may be a device with an inlet and outlet, preferably a cylindrical device with an inlet and an outlet. To at least a portion of said device, electric pulses may be applied, for example using electrodes as described below. The inlet and/or the outlet may be provided with connecting units such as valves, which allow the controlled and/or combined application of separate liquids into said device, and/or the separation of a treated sample into different separate samples.

For the method of the present invention, only small sample sizes are required. For example, a few ml, preferably 1-5 ml, or even a few µl, preferably 100-500 µl of a sample with cellular material are necessary for each treatment under varying conditions.

According to this embodiment of the present invention, said treatment area is a portion of the single-flow device that is located between electrodes. In other words, electrodes and preferably two electrodes are provided, preferably parallel to one another, alongside the single-flow device, so that an area between these electrodes is formed.

The two or more, preferably two, electrodes emit electrical pulses into the portion of the single-flow device that is located between said electrodes. Alternatively, two plates e.g. of a capacitor may be used for this purpose. The electrodes are arranged such that said portion of the single-flow device can be penetrated by the electric pulses emitted by said electrodes and an electric field resulting therefrom. Preferably, said electrodes or plates e.g. of a capacitor are arranged parallel to each other, opposite each other having a distance suitable for the generation of adequate electric fields. Such arrangements are well known and need not be explained in detail here.

Said electrodes or plates e.g. of a capacitor are electrically connected with a unit for generating electric pulses. Such electrical connections are known and do not have to be discussed in detail here. Such units for generating electric pulses are also generally known. By way of example, cable pulse generators, semiconductor-based pulse generators, or relaxation oscillators can be mentioned.

Preferably, said system may be a bioreactor. A bioreactor is generally known in the art. A bioreactor is a device or system that supports a biologically active environment. Preferably, a bioreactor is a vessel in which a (bio)chemical process can be carried out which involves organisms or biochemically active substances derived from such organisms. This process can either be aerobic or anaerobic, for example fermentation. Conventional bioreactors are often made of stainless steel.

According to a preferred embodiment of the present invention, said system may comprise one or more lines arranged at said single-flow device. According to the present invention, any line conventionally used for transporting a fluid may be used. Preferably, the lines are pipes, for example made from stainless steel, tubes or hoses.

The electric field to be applied to the treatment area must be characterized such that it provides for the desired effect, e.g. that it stimulates the growth of the treated cells. Corresponding electric fields are known from the prior art, for example from EP-2 308 969 B1. According to the present invention, an electric field generated from such electric pulses can be used, such that a voltage increase takes place between the two electrodes or plates of a capacitor of the device of 10% to 90% of a target voltage of the electric pulses within a period of 0.1 ns to 1000 ns, the electric pulses have a pulse duration of 5 ns to 50000 ns, and the electric pulses, upon reaching the target voltage, have an electric field strength of 0.5 kV/cm to 100 kV/cm.

According to a preferred variant of the present invention, the step of treating samples comprising cellular material under different conditions involves a variation of the electric field described above. Each treatment condition is characterized by s specific pulse duration, a specific electric field strength, and a specific number of applied pulses. Merely by way of example, a variation as follows may be conducted:
1. condition: 5 kV/cm, 100ns, 2-10 pulses
2. condition: 10 kV/cm, 100ns, 2-10 pulses
3. condition: 20 kV/cm, 100ns, 2-10 pulses.

In other words, a first sample of a cellular material may be introduced into the treatment area, and for example the above described first condition may be applied. Subsequently, a second sample of a cellular material may be introduced into the treatment area, and for example the above described second condition may be applied. Subsequently, a third sample of a cellular material may be introduced into the treatment area, and for example the above described third condition may be applied. In this way, three samples can be obtained in a time-efficient manner, which have been treated under different conditions. Needless to say, the above number of different samples, as well as the above number and type of different treatment conditions are not limiting. Step a) can generally be applied to cellular material derived from a single source or multiple sources that are exposed to conditions and procedures as described in step a).

According to another variant of the first embodiment of the present invention, in step a) the sample comprising cellular material is treated under one condition. Said one condition may be one of the conditions described above, e.g. one of conditions 1 to 3 described above. Also the treatment area may be as described above.

Said variant differs in that from said treatment of a sample comprising cellular material under one condition, suitable conditions may be identified using a machine-learning module. This will be discussed below with respect to step b).

Accordingly, in a preferred embodiment of a subsequent step b) of the method of the present invention, the result of the treatment in step a) under one applied condition is analysed using a machine-learning module.

The machine-learning module can e.g. be based on supervised or unsupervised learning structures, as known in the art.

Preferably, the machine-learning module can e.g. convert the measured parameters into a sequence of assigned binary input codes and process the sequence of the assigned binary input codes by applying a maximum likelihood parameter estimation for the training of a multi-dimensional data structure of the machine-learning module with the variable hidden Markov model.

The hidden Markov model is known and does not have to be described in detail here. Generally, it is used to estimate an unknown condition (hidden variables) from an observed condition.

According to a preferred variant of the present invention, the above described binary input codes are processed by applying a maximum likelihood parameter estimation. With thus obtained variable hidden Markov parameters, the training of a multi-dimensional data structure of the machine-learning module is conducted, wherein the elements of a sequence of storable parameter states of the Markov chain are assumed to be independent measurements of each other, and wherein the model parameters of the multi-dimensional data structure are varied by maximizing the multiplied product of the probabilities in order to obtain the trained model parameters of the multi-dimensional data structure.

The model parameters of the multi-dimensional data structure can e.g. be iteratively varied until a predefined convergence threshold is triggered. For determining said threshold value of a score indicating or providing a measure for the optimization of the operational parameters, an averaging process can e.g. be applied based on the different pattern parameters of the sensory and/or measuring data of an identified time frames.

In a variant of said embodiment of the invention, the sensitivity of the chosen operational parameters can e.g. be automatically tuned based on dynamic adjustments of the threshold value. This embodiment variant has inter alia the advantage, that the convergence speed by training the variable hidden Markov model parameters of the multi-dimensional data structure can be optimized.

In another preferred embodiment of a subsequent step b) of the method of the present invention, the results obtained in step a) under different conditions are analysed with respect to the desired characteristics to be achieved, e.g. stimulation of cell growth. The desired characteristics to be achieved are not particularly limited. Any characteristic that plays a role for achieving a certain effect of the treated material may be considered. For example, the stimulation of cell growth and/or the stimulation of the generation of cellular compounds may be suitable desired characteristics.

According to a preferred variant, step b) is performed directly after step a). In other words, after the sample has left the treatment area, analysis is directly performed. The analytical method may be chosen depending on the desired characteristics to be achieved. For example, for analysing characteristics corresponding to cell growth and/or cellular compound production, flow cytometry or impedance spectroscopy may be used. Such analytical methods are known and need not be discussed here in detail.

Analysis may be performed online, if possible, or by taking a probe and analysing said probe off-line.

According to another variant of the first embodiment, step b) is performed after the treated sample has been cultivated in a cultivation system, wherein said cultivation system comprises at least one cultivation container, for example an Erlenmeyer flask. In this variant, the sample is thus allowed some time in the cultivation system for manifestation of the desired characteristics to be achieved, before an analysis is carried out. The duration of the time for manifestation of the desired characteristics to be achieved is dependent on the kind of said characteristics and known to a skilled person. For example, the sample may be given a manifestation time of 0.5-10 days, preferably 2-5 days.

In this variant, differently treated samples may be divided onto different cultivation systems by means of suitable connecting units such as valves, which allow the separation of a treated sample into different separate samples to be cultivated in separate cultivation systems.

Analysis of the desired characteristics to be achieved is carried out in a unit for analysing the results of a treatment under at least one treatment condition, preferably two or more varying treatment conditions, and identifying suitable conditions. Such a unit is known and typically is a processing unit such as a computer. If the variant involving treatment under one condition is used, the unit comprise a machine-learning module as described above.

Typically, said unit for analysing the results of a treatment under at least one treatment condition, preferably two or more varying treatment conditions, and identifying suitable conditions receives data from a component of an analyser unit, such as a detector. Once the data of one or several analytical steps with one or varying probes have been collected by the unit for analysing the results of a treatment under at least one treatment condition, preferably two or more varying treatment conditions and identifying suitable conditions, said unit may perform a comparison and therewith determine suitable treatment conditions. Said suitable treatment conditions can subsequently be employed in the actual treatment of cellular material. Said actual treatment may involve scale-up treatments for industrial applications. Alternatively, said unit may analyse the result of a treatment under one condition using a machine-learning module, as described above. Preferably, said unit comprises a machine-learning module that applies a variable hidden Markov model, as described above.

According to the first embodiment of the present invention, the method may be performed as a method selected from the groups consisting of a batch method, a fed-batch method, and a continuous method. These different kinds of methods are known and do not have be explained here.

According to a second preferred embodiment of the present invention, step a) of the above method, i.e. the step of treating a sample comprising cellular material under different conditions, is performed in a high-throughput system comprising a treatment area with a plurality of varying treatment conditions.

A high-throughput system is a system that is suitable for high-throughput testing appliances. Preferred are multi-well plates, i.e. a plate having a specific number of wells in which different tests can be carried out. Such multi-well plates are known and may comprise, for example, 4 to 1536 wells. As an example, 6-, 12-, 24-, 48-, 96-, 384- and 1536-well plates are mentioned. Preferably, according to this second embodiment said plurality of varying treatment conditions are provided in wells of a well-plate.

Typical volumes being analysed in multi well plates are in the range of about 1 to 2 ml, preferably 100 nl to about 500 µl, more preferably of about 200 nl to about 20 µl.

In the wells of a multi-well plate, suitable conditions for cellular material may be provided which e.g. sustain cellular growth. Such conditions are known and may involve the use of three-dimensional matrices such as Matrigel^{®} or synthetic matrices as described in e.g. WO 2014/180970 A1, as well as of culture media containing e.g. growth factors.

According to a preferred variant of the second embodiment of the present invention, cellular material is provided in wells of a multi-well plate, preferably in the form of a suspension. Subsequently, said cellular material in those wells is treated under varying treatment conditions. Said varying treatment conditions may be applied by at least one unit.

Said unit for applying said plurality of varying treatment conditions may be a movable component comprising electrodes, preferably parallel and/or serial to one another. Said unit may be moved into a well of a multi-well plate. When these electrodes and preferably two electrodes enter the well, an area between these electrodes is formed.

The two or more, preferably two, electrodes emit electrical pulses into the portion of the well that is located between said electrodes. Alternatively, two plates e.g. of a capacitor may be used for this purpose. The electrodes are arranged such that said portion of the well can be penetrated by the electric pulses emitted by said electrodes and an electric field resulting therefrom. Preferably, said electrodes or plates e.g. of a capacitor are arranged parallel to each other, opposite each other having a distance suitable for the generation of adequate electric fields. Such arrangements are well known and need not be explained in detail here.

Said electrodes or plates e.g. of a capacitor are electrically connected with a unit for generating electric pulses. Such electrical connections are known and do not have to be discussed in detail here. Such units for generating electric pulses are also generally known. By way of example, cable pulse generators, semiconductor-based pulse generators, or relaxation oscillators can be mentioned.

Movement of said unit for applying said plurality of varying treatment conditions may be performed by any suitable known means. For example, a connecting arm operated by an electric motor and capable of performing horizontal and vertical movements may be used.

If only one unit for applying said plurality of varying treatment conditions is used, treatment of the cellular material in the various wells is performed sequentially, by moving said unit into one well after another and performing the different treatment operations in each well.

According to another variant, a plurality of units for applying said plurality of varying treatment conditions may be used which enable said treatment of the cellular material in the various wells to be at least partially performed in parallel and/or serial. Typically, the wells of a multi-well plate are arranged in rows, e.g. in rows of each 8 wells. If an array of 8 units for applying said plurality of varying treatment conditions is provided, in all wells of a single or multiple row treatment may be performed in parallel and/or serial. Such array of units may be realized in a conventional manner, by combining said units at an appropriate distance to one another in a suitable support.

Like in the variant with a single unit, the units of such an array are provided with electrodes and are operated in the same manner.

It is understood that each possible variation of arrangements of units for applying said plurality of varying treatment conditions can be used. The present invention is not limited to the variants specifically described above.

The electric field to be applied to a well of a multi-well plate corresponds to the electric field described above for the first embodiment. Also, the variation of the treatment conditions in the high-throughput screening system may be performed as described above for the first embodiment.

In the variant where only one unit for applying said plurality of varying treatment conditions is used, said unit enters each well sequentially. In different wells, different variations of the electric field may be applied, so that cellular material in different wells may be treated under different conditions.

In the variant where an array of units for applying said plurality of varying treatment conditions is used, said units may apply the same electric field or variations of the electric field to different wells. This can be carried out with the use of a suitable known controlling device which may provide different units with different electric pulses.

Accordingly, a part or preferably all of the plurality of varying treatment conditions are carried out in parallel and/or serial.

According to another preferred variant of the second embodiment of the present invention, electrodes are provided above and below the high-throughput system, preferably the wells of a multi-well plate. Thus, instead of the unit for applying said plurality of varying treatment conditions described above, said treatment conditions are provided here to the high-throughput system by means of said electrodes.

As described above, also said electrodes, preferably two electrodes, emit electrical pulses into the portion of the high-throughput system that is located between said electrodes. Alternatively, two plates e.g. of a capacitor may be used for this purpose. The electrodes are arranged such that said portion of the high-throughput system, preferably the wells of a multi-well plate, can be penetrated by the electric pulses emitted by said electrodes and an electric field resulting therefrom. Preferably, said electrodes or plates e.g. of a capacitor are arranged parallel to each other, opposite each other having a distance suitable for the generation of adequate electric fields. Such arrangements are well known and need not be explained in detail here.

Said electrodes or plates e.g. of a capacitor are electrically connected with a unit for generating electric pulses. Such electrical connections are known and do not have to be discussed in detail here. Such units for generating electric pulses are also generally known. By way of example, cable pulse generators, semiconductor-based pulse generators, or relaxation oscillators can be mentioned.

It is possible to arrange a pair of electrodes above and below each well of a multi-well plate, respectively. However, it is preferred to provide two electrodes consisting of an array of portions that may serve as electrodes above and below each well of a multi-well plate. Said electrodes may be operated like the units for applying said plurality of varying treatment conditions described above.

According to a preferred embodiment, said electrodes are transparent.

Analysis of the cellular material in the different wells may be performed as described above for the first embodiment. That is, analysis of the desired characteristics to be achieved is carried out in a unit for analysing the results of a treatment under at least one treatment condition, preferably two or more varying treatment conditions and identifying suitable conditions. Such a unit is known and typically is a processing unit such as a computer.

Typically, said unit for analysing the results of a treatment under at least one treatment condition, preferably two or more varying treatment conditions and identifying suitable conditions receives data from a component of an analyser unit, such as a detector. Once the data of several analytical steps with varying probes have been collected by the unit for analysing the results of a treatment under at least one treatment condition, preferably two or more varying treatment conditions and identifying suitable conditions, said unit may perform a comparison and therewith determine suitable treatment conditions. Said suitable treatment conditions can subsequently be employed in the actual treatment of cellular material. Said actual treatment may involve scale-up treatments for industrial applications.

According to a preferred variant of the second embodiment, said analyser unit may perform the analysis of the cellular material directly in the wells of said well-plate. Suitable analyser systems for high-throughput systems are known.

According to another preferred embodiment, from the analysis of step c) suitable conditions are stored in a database and used to determine the initial condition or set of conditions used in a subsequent treatment of cells.

The present invention is furthermore related to a system for performing the method described above, comprising a treatment area to which at least one treatment condition, preferably two or more varying treatment conditions can be applied, and a unit for analysing the results of a treatment under at least one treatment condition, preferably two or more varying treatment conditions and identifying suitable conditions.

As described above, said treatment area may comprise a single flow-through unit to which varying treatment conditions can be applied. Said embodiment of the system of the present invention may be used for performing the method according to the first embodiment described above.

In this case, the system may further comprise a cultivation system, wherein the cultivation system comprises at least one cultivation container, for example an Erlenmeyer flask, for cultivation of a treated probe before analysis.

As also described above, said treatment area may alternatively comprise a high-throughput system, preferably a well-plate with wells, in which a plurality of varying treatment conditions can be performed. Said embodiment of the system of the present invention may be used for performing the method according to the second embodiment described above.

In this case, the system may further comprise at least one unit for applying said plurality of varying treatment conditions either sequentially or in parallel to samples comprising cellular material.

Alternatively, the system may comprise electrodes, preferably transparent electrodes, that are integrated into the high-throughput system and which allow for parallel application of said plurality of varying treatment conditions to the high-throughput system.

Details on the system of the present invention and its different variants have been discussed above with respect to the first and second embodiment of the method of the present invention.

With the method and system of the present invention, typically biological material in the form of a suspension is treated.

According to the present invention, said system is suitable for supporting medical, environmental, food applications, and bio-based industries (including yeast, lactobacilli, algae, and cell tissue production systems, in particular including targeted inactivation, the extraction of bioactive compounds, and the stimulation of cell growth and/or cellular compounds, by selecting suitable treatment conditions. These applications are described in Buchmann L and Mathys A (2019), Perspective on Pulsed Electric Field Treatment in the Bio-based Industry, Front. Bioeng. Biotechnol. 7:265, doi: 10.3389/fbioe.2019.00265.

According to the present invention, electric pulses are applied to a cell material located in the treatment area of the system according to the present invention. As a result, the cell material is subjected to the electric pulses and treated.

According to the present invention, basically any material composed of at least one cell, that is, both eukaryotic and prokaryotic cells, can be used as the cell material to be treated. The cell material can be unicellular or multicellular organisms. Examples would be bacteria, yeasts, microalgae, plant cells, and fungal cells or their spores, mycelia, seeds or seedlings and somatic animal cells or germ cells and mammalian cells. Furthermore, multicellular tissues such as meristems in plants and epithelial or connective tissue in humans or animals can be treated.

The cell material is usually (but not necessarily) isolated and/or purified in a known manner before being treated according to the invention. Preferably, the cell material can already be propagated in a known manner in suitable and known culture media to a desired degree before the treatment according to the invention.

The cell material is preferably suspended in an electrically conductive liquid prior to the treatment according to the invention. Electrically conductive liquids are well known. According to the invention, it is necessary to use electrically conductive liquids which have no adverse effects on cell viability, that is, in particular, are non-toxic. According to the invention, water is preferably used as the electrically conductive liquid, wherein the water can be adjusted to a desired pH value by means of suitable and known additives. According to the invention, a pH value in the range of 6.0 to 14.0, preferably 7 to 12 is preferred. According to another preferred embodiment of the invention, macro- and micronutrients as well as trace elements can be added for supporting cultivation and/or proliferation of the cells. Such macro- and micronutrients as well as trace elements are known and can be selected by a skilled person depending on the kind of cells.

According to the present invention, the suspensions described above can be prepared in a conventional manner and stored until treatment. However, the suspensions can also be provided immediately before the treatment according to the invention.

The present invention further relates to the use of the system according to the present invention described herein for medical, environmental, food applications, and bio-based industries (including yeast, bacteria, microalgae, as well as plant or animal cells and cell tissue production systems, in particular targeting inactivation, the extraction of bioactive compounds, and/or the stimulation of cell growth and/or cellular compounds. These applications are described in Buchmann L and Mathys A (2019), Perspective on Pulsed Electric Field Treatment in the Bio-based Industry, Front. Bioeng. Biotechnol. 7:265, doi: 10.3389/fbioe. 2019.00265.
With the suitable conditions identified with the method of the present invention, a method for treating cells for stimulating cell growth may be performed, as e.g. described in EP-2 308 969 B1, by subjecting cell material to electric pulses. This method may be performed in any known suitable device. Preferably, a device as described in one of applicant's co-pending applications "Device for treating cells" (PCT/EP2021/052665), "Non-contact device for treating cells" (PCT/EP2021/052667), "Modified treatment chamber for treating cells" (PCT/EP2021/052674), or "Device for treating cells in a bypass" (EP20208112.1) may be used.

Thus, the present invention is also related to a method for treating cells for targeted inactivation, the extraction of bioactive compounds, and the stimulation of cell growth and/or cellular compounds, comprising the steps of
- identifying suitable treatment conditions in a method as described above,
- introducing cell material into a treatment space, and
- applying electric pulses corresponding to the identified treatment conditions to said treatment space, wherein said electric pulses penetrate the treatment space.

The present invention is explained below by way of non-limiting examples and figures.
- Fig. 1: shows a schematic representation of a first embodiment of the system of the present invention
- Fig. 2: shows a schematic representation of a variant of the first embodiment of the system of the present invention that further comprises a cultivation system
- Fig. 3: shows a schematic representation of a second embodiment of the system of the present invention
- Fig. 4: shows a schematic representation of a variant of the second embodiment of the system of the present invention.

In the figures, same references numbers designate the same components.

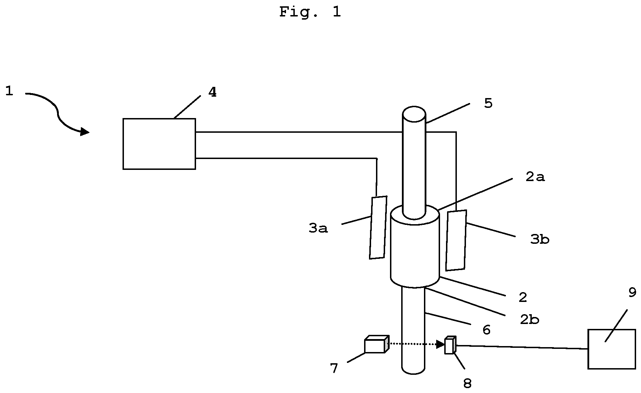

In Fig. 1, a schematic representation of a first embodiment of the system 1 of the present invention is shown. Said system 1 comprises a unit 4 for generating electric pulses, which by means of electrodes 3a, 3b are emitted into a treatment area 2. Said treatment area 2 is realized here in the form of a single flow-through unit.

Said treatment area 2 comprises an inlet 2a to which a line 5 (here a pipe) is connected that leads the cellular material to be treated into the treatment area.

Said treatment area 2 further comprises an outlet 2b to which a line 6 (here a pipe) is connected for leading the treated cellular material out of the treatment area.

If desired, units for regulating the flow (not shown) may be provided. As an example, conventionally used valves or locks may be mentioned.

The two electrodes 3a, 3b emit electrical pulses into the portion of the single-flow device that is located between said electrodes, as described above.

After the treated cellular material has left the treatment area 2, it is analysed for desired characteristics to be achieved. In the embodiment shown in Fig. 1, an analyser unit comprising a measurement source 7 such as a laser and a detector 8 is provided downstream the treatment area 2 for performing an on-line analysis. Alternatively, an opening for taking a probe could be provided. The taken probe is then analysed off-line in a suitable analyser unit.

The analyser unit is connected with a unit 9 for analysing the results of a treatment under varying treatment conditions and identifying suitable conditions. As described above, this may be a processing unit such as a computer.

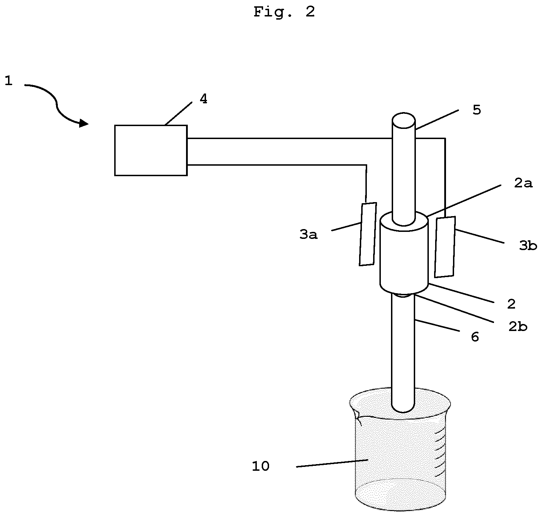

In Fig. 2, a schematic representation of a variant of the first embodiment of the system 1 of the present invention is shown. The variant according to Fig. 2 differs in that the analytical step is not performed directly after treatment of the cellular material in the treatment area 2. Rather, the treated cellular material is led into a cultivation system 10, such as an Erlenmeyer flask. In said cultivation system 10, the treated cellular material is kept for a time sufficient for manifestation of the desired characteristics to be achieved. This time is dependent on the kind of said characteristics and known to a skilled person. For example, the sample may be given a manifestation time of 0.5-10 days, preferably 2-5 days.

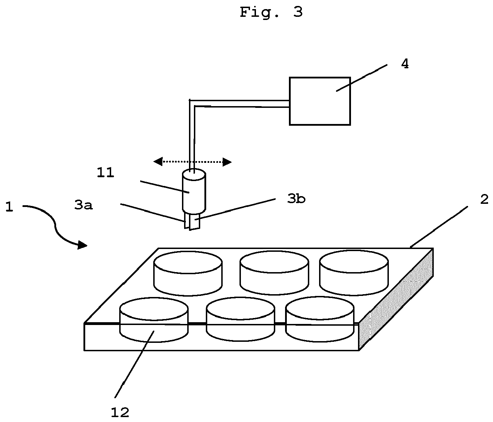

In Fig. 3, a schematic representation of a second embodiment of the system 1 of the present invention is shown. In this second embodiment of the system 1 of the present invention, the treatment area 2 is a high-throughput system in the form of a multi-well plate with wells 12. In Fig. 2, merely for the sake of illustration a plate with 6 wells 12 is shown.

The cellular material to be treated is provided in the wells 12. A unit 11 for applying said plurality of varying treatment conditions is provided on a connecting arm (not shown) that moves said unit 11 from one well to the other. Treatment is conducted here in a sequential manner. In Fig. 3, the analytical unit as well as the unit 9 for analysing the results of a treatment under varying treatment conditions and identifying suitable conditions is not shown.

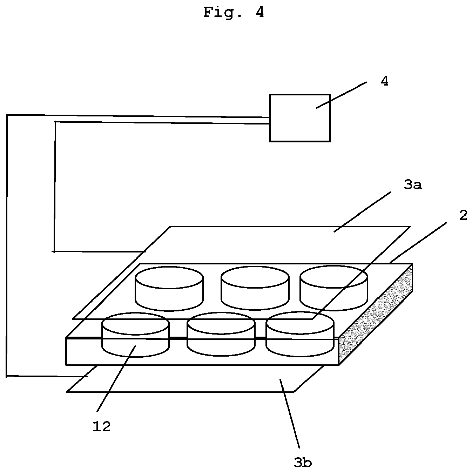

In Fig. 4, a schematic representation of a variant of the second embodiment of the system of the present invention is shown.

Here, transparent electrodes 3a, 3b are provided above and below the treatment area 2 being a high-throughput system in the form of a multi-well plate with wells 12, instead of the unit 11 of the variant of Fig. 3. In Fig. 4, the analytical unit as well as the unit 9 for analysing the results of a treatment under varying treatment conditions and identifying suitable conditions is not shown.

## Claims

1. A method for identification of optimized conditions for treating cells with electric pulses for targeted inactivation, the extraction of bioactive compounds, and the stimulation of cell growth and/or cellular compounds, comprising the steps of:
a) treating samples comprising cellular material with at least one condition, preferably two or more different conditions,
b) analysing the results of the treatment in step a) for the applied condition(s),
c) identifying suitable conditions from the analysis of step b) .

2. The method according to claim 1, wherein from the analysis of step c) suitable conditions are stored in a database and used to determine the initial condition or set of conditions used in a subsequent treatment of cells.

3. The method according to any of claims 1 to 2, wherein step a) is performed in a system (1) where the samples flow through a treatment area (2) to which varying treatment conditions are applied, and for each of the applied treatment condition a probe is analysed in step b).

4. The method according to claim 3, wherein step b) is either performed directly after step a), for example with flow cytometry or impedance spectroscopy, or is performed after the treated sample has been cultivated in a cultivation system (10), wherein the cultivation system comprises at least one cultivation container, for example an Erlenmeyer flask.

5. The method according to any of claims 1 to 4, wherein in step a) the samples comprising cellular material is treated with one condition, and the analysis in step b) is performed with a machine-learning module, preferably a machine-learning module applying a variable hidden Markov model.

6. The method according to any of claims 1 to 4, wherein step a) is performed in a high-throughput system comprising a treatment area (2) with a plurality of varying treatment conditions, wherein said plurality of varying treatment conditions are provided in wells (12) of a well-plate and wherein preferably the plurality of varying treatment conditions are carried out in parallel and/or serial.

7. The method according to claim 6, wherein step b) is performed directly in the wells (12) of said well-plate.

8. A method for treating cells for targeted inactivation, the extraction of bioactive compounds, and the stimulation of cell growth and/or cellular compounds, comprising the steps of
- identifying suitable treatment conditions in a method according to any of claims 1 to 7,
- introducing cell material into a treatment space, and
- applying electric pulses corresponding to the identified treatment conditions to said treatment space, wherein said electric pulses penetrate the treatment space.

9. A system (1) for performing the method according to any of claims 1 to 7, comprising a treatment area (2) to which at least one treatment condition, preferably two or more varying treatment conditions can be applied, and a unit (9) for analysing the results of a treatment under at least one treatment condition, preferably two or more varying treatment conditions and identifying suitable conditions.

10. The system according to claim 9, wherein said treatment area (2) comprises a single flow-through unit to which varying treatment conditions can be applied.

11. The system according to claim 9 or 10, wherein the unit (9) for analysing the results of a treatment under at least one treatment condition comprises a machine-learning module, preferably a machine-learning module that applies a variable hidden Markov model.

12. The system according to any of claims 9 to 11, further comprising a cultivation system (10), wherein the cultivation system comprises at least one cultivation container, for example an Erlenmeyer flask, for cultivation of a treated probe before analysis.

13. The system according to claim 9, wherein said treatment area (2) comprises a high-throughput system, preferably a well-plate with wells (12), in which a plurality of varying treatment conditions can be performed.

14. The system according to claim 13, wherein at least one unit (11) is provided for applying said plurality of varying treatment conditions either sequentially or in parallel to samples comprising cellular material.

15. The system according to claim 13, wherein electrodes (3a, 3b), preferably transparent electrodes, are integrated into the high-throughput system, allowing for parallel and/or serial application of said plurality of varying treatment conditions to the high-throughput system.
